# EUROPEAN PATENT SPECIFICATION

(11) **EP 2 694 159 B1**
(45) Date of publication and mention of the grant of the patent: **05.10.2016**
(21) Application number: 12764427.6
(22) Date of filing: 27.03.2012
(51) Int. Cl.: A61N 5/06

(54) **LIGHT AND VIBRATION TREATMENT DEVICE**
LICHT- UND VIBRATIONS-BEHANDLUNGSVORRICHTUNG
DISPOSITIF DE TRAITEMENT PAR LA LUMIERE ET VIBRATION

(30) Priority: 01.04.2011 US 201161470723 P
(43) Date of publication of application: 12.02.2014
(73) Proprietor: Syneron Beauty Ltd, 20692 Yoqneam Illit (IL)
(72) Inventor: KENNEDY, John, Duntroon, Ontario LOM 1H0 (CA); YU, Jinxin, Mississauga, Ontario L5L 1G4 (CA)
(74) Representative: ZBM Patents - Zea, Barlocci & Markvardsen
(86) International application number: PCT/IL2012/000134
(87) International publication number: WO 2012/131672

(56) References cited:
- WO-A1-2006/012752
- US-A- 5 176 130
- US-A1- 2004 000 316
- US-A1- 2004 147 984
- US-A1- 2006 226 378
- US-A1- 2007 198 004
- US-A1- 2007 260 295
- US-A1- 2009 048 557
- US-B2- 7 014 639
- US-B2- 7 896 908

## Description

### FIELD

This specification relates to treatment devices and in particular to portable hand held devices for treating conditions, such as skin conditions, through the application of light, heat and/or vibration therapies.

### BACKGROUND

A variety of skin conditions may be treated through the topical application of therapeutic substances and controlled exposure to specific wavelengths and intensities of light (electromagnetic radiation). Treatment of such skin conditions may be enhanced by mechanical stimulation such as vibratory stimulation or the application of heat to the desired treatment area.

### SUMMARY

The present disclosure refers to a skin treatment device as defined in claim 1.

In one aspect there is provided a skin treatment device including a potting containing one or more light sources for emitting electromagnetic radiation having desired characteristics for treating one or more skin conditions, a skin contacting surface disposed at one end of the potting, the skin contacting surface being adapted for transmitting electromagnetic radiation emitted from the at least one light source to a user's skin and to transfer heat generated by operation of the at least one light source to the user's skin, a vibrator directly or indirectly contacting the other end of the potting, the vibrator being adapted for causing the skin contacting surface to vibrate and a control assembly for controlling operation of the light source and the vibrator.

In another aspect there is provided a skin treatment device, including a body having a treatment head at one end and a dispenser head at another end, the treatment head including one or more light sources for emitting electromagnetic radiation having desired characteristics for treating one or more skin conditions and to transfer heat generated by operation of the at least one light source to the user's skin, the dispenser head including a dispensing assembly for dispensing a desired substance for use in treating one or more skin conditions and a control assembly for controlling operation of the light source.

In yet another aspect there is provided a treatment head for a skin treatment device, the treatment head including a potting containing at least one light source for emitting electromagnetic radiation having desired characteristics for treating one or more skin conditions, a skin contacting surface disposed at one end of the potting, the skin contacting surface being adapted for transmitting electromagnetic radiation emitted from the at least one light source to a user's skin and to transfer heat generated by operation of the at least one light source to the user's skin, a vibrator directly or indirectly contacting the other end of the potting, the vibrator being adapted for causing the skin contacting surface to vibrate.

Other aspects and features of the teachings disclosed herein will become apparent, to those ordinarily skilled in the art, upon review of the following description of the specific examples of the specification.

### DRAWINGS

The drawings included herewith are for illustrating various examples of articles, methods, and apparatuses of the present specification and are not intended to limit the scope of what is taught in any way. For simplicity and clarity of illustration, where considered appropriate, reference numerals may be repeated among the drawings to indicate corresponding or analogous elements.
Figure 1 is perspective view simplified illustration of a treatment device in accordance with one example;
Figures 2A and 2B are perspective sectional view simplified illustrations of the device of Figure 1 as viewed along lines W-W;
Figure 3 is a perspective view simplified illustration of the device of Figure 1 showing the dispenser head removed from the remainder of the device;
Figure 4 is an exploded perspective view simplified illustration of the device of Figure 1;
Figure 5 is an enlarged sectional view simplified illustration of the treatment head for the device of Figure 1 as viewed along lines W-W
Figure 6 is a block diagram of certain functional components for the device of Figure 1;
Figure 7 is a sectional view simplified illustration of a treatment head of a treatment device in accordance with another example;
Figure 8 is a sectional view simplified illustration of a treatment head of a treatment device in accordance with another example; and
Figures 9A - 9F are plan views simplified illustrations of more examples of skin contacting surfaces for a treatment device.

### DESCRIPTION

The claimed device is not limited to devices having all of the features of any one device or method described below or to features common to multiple or all of the devices described below. The claimed device may include a combination or subcombination of the device elements or method steps described below. It is possible that a device or method described below is not an example of the claimed device. The applicant(s), and/or owner(s) reserve all rights in any device or method described below that is not claimed in this document and do not abandon, disclaim or dedicate to the public any such device by its disclosure in this document.

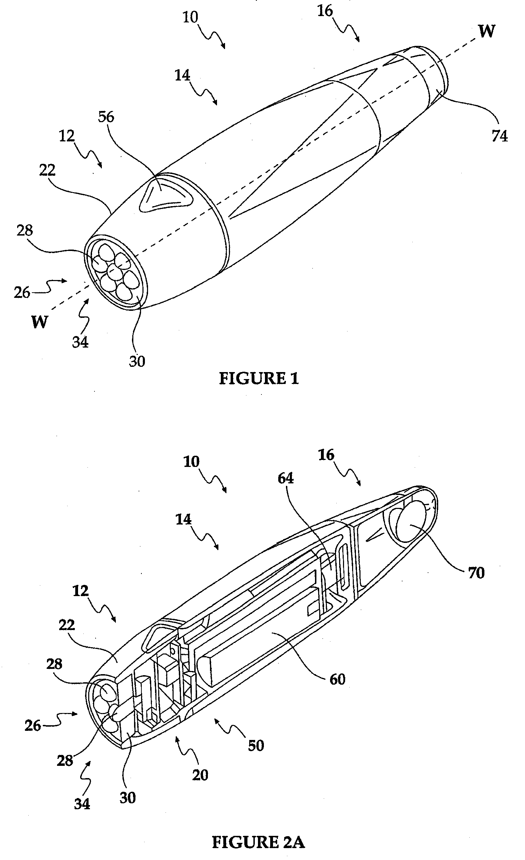

A treatment device 10 in accordance with an example is shown generally in Figure 1. The device includes a treatment head 12, a body 14 and a dispenser head 16.

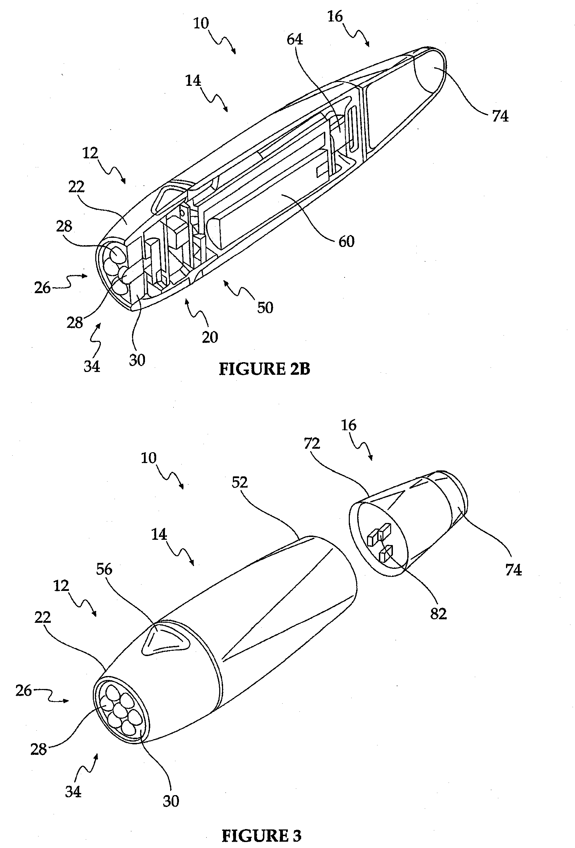

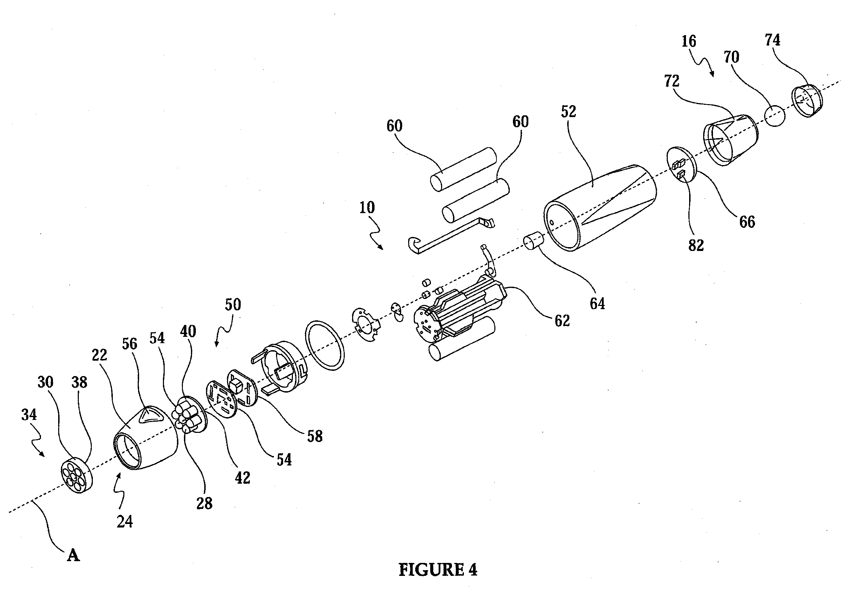

Treatment head 12 includes a treatment assembly 20 (Figs. 2A and 2B) disposed in a treatment head housing 22. Treatment head housing 22 defines a cavity 24 (Fig. 4) for receiving treatment assembly 20 and an aperture 26 at one end. Treatment assembly 20 includes at least one light source 28 that is at least partially encapsulated in a potting 30. Light source 28 can be commonly a light emitting diode (LED), such as a chip-in-board or surface mount LEDs, and more commonly an encapsulated LED.

Light source 28 commonly operates in the range of 400-1500 nm. More commonly light source 28 operates in the range of 400-450 nm for the treatment of acne (and most commonly 414 nm), 600-1000 nm for skin rejuvenation (and most commonly 660 nm) and 800-1000 nm for micro-circulation stimulation (and most commonly 870 nm). The energy output for treatment using light source 28 is commonly in the range of 5-15 J/cm². For a blue light (414nm) treatment the energy output is commonly about 6 J/cm². For a treatment time of 120 seconds, the power density is commonly 50 mW/cm². On some other wavelengths, the energy outputs may be in the range of 7 J/cm² and 8.4 J/cm² assuming the same amount of treatment time. And, if a single-point light source 28 is used, an energy output of 15 J/cm² or more may be applied.

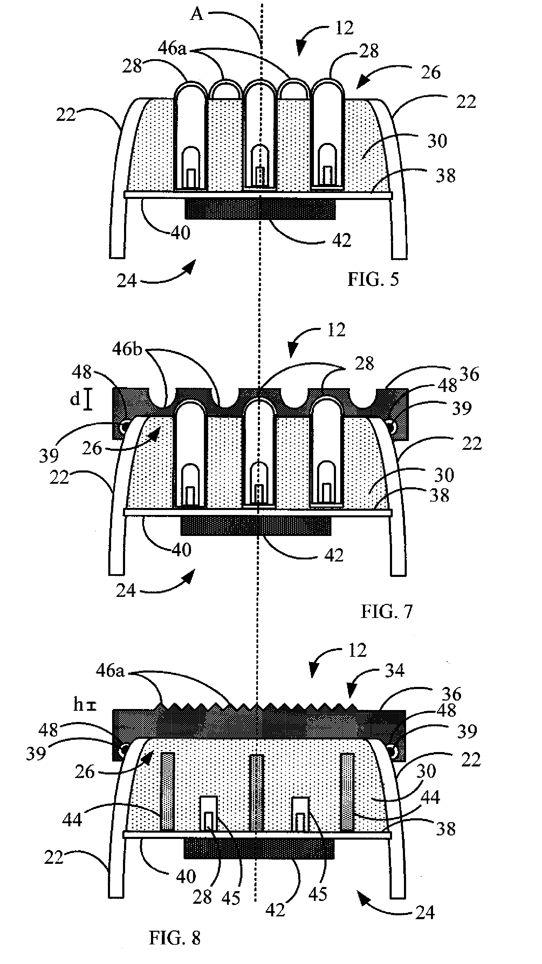

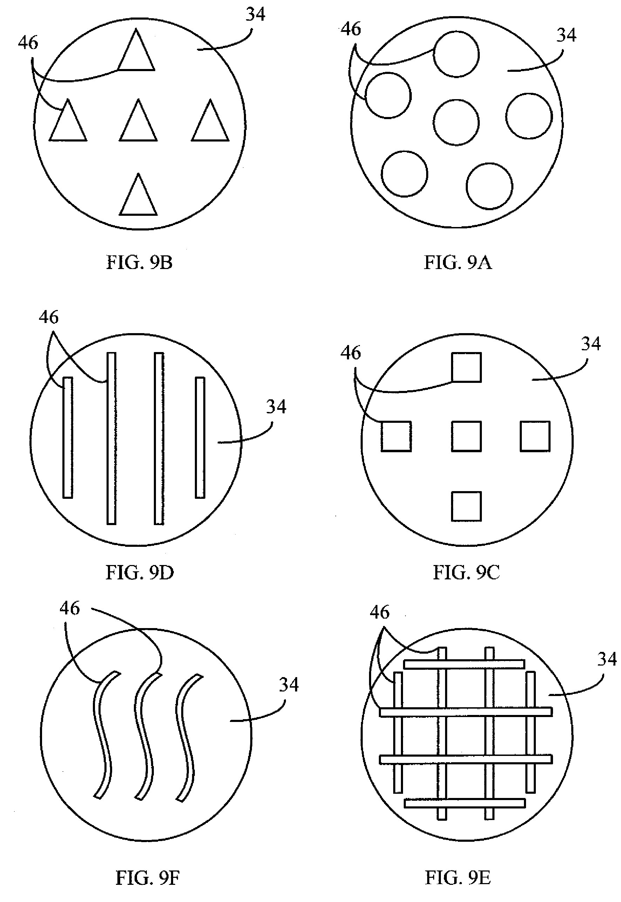

A plurality of light sources 28 are commonly encapsulated within potting 30 such as, for example purposes only, a cluster of seven or any other number of encapsulated LED light sources evenly distributed in a circular arrangement as shown in Figures 1-5. Other configurations of shapes and arrangements may be employed such as shown in Figures 7-9. Potting 30 for example purposes only, can be a transparent epoxy potting that encases a substantial portion of light source 28. When light source 28 comprises one or more encapsulated LEDs, such a transparent epoxy potting 30 commonly has a refractive index that is similar to the refractive index of the epoxy material used to encapsulate the LEDs. An acrylic epoxy potting 30 could be selected for instance due to its refractive index of approximately 1.5 at 20 degrees Celsius which is similar to the refractive index range of 1.47-1.52 for the LED epoxy encapsulation.

In one example of device 10 as shown in Figures 1-5, potting 30 is disposed in a cavity 24 (Fig. 4), generally flush with aperture 26. The tips of the encapsulated LED light sources 28 protrude a short distance (commonly between 0.5-5 mm) from aperture 26. The exposed face of potting 30 and the protruding tips of encapsulated LEDs together define a skin contacting surface 34 of treatment assembly 20. Skin contacting surface 34 may alternatively be defined by a faceplate 36 (Figs. 5, 7 and 8), commonly formed of a transparent epoxy material, disposed at the end of treatment head 12 and adapted for transferring light, heat and vibration energy to the skin such as shown in Figures 7-9. Faceplate 36 may be removably connected to the end of treatment head 12 by a connection 48 (Figs. 7 and 8) such as a snap fit over a bead 39 on treatment head housing 22, a threaded connection (not shown) or other suitable means of removable connection. The removable connection allows a user to interchangeably fit a variety of faceplates according to a desired treatment.

The remainder of potting 30 is disposed within cavity 24 (Figs. 4, 5, 7 and 8) and includes a non-skin contacting surface 38, disposed on a side of potting 30 opposite to skin contacting surface 34 and connected to a first printed circuit board (PCB) 40. First PCB 40 includes a plurality of vent holes (not shown) that allow air to escape when the molten material for forming potting 30 is disposed within treatment head housing 22.

Potting 30 acts to transfer heat generated from light sources 28 to skin contacting surface 34. It has been found that encapsulated LED light sources 28 generate sufficient heat, upon transfer through skin contacting surface 34, to aid in the treatment of a variety of skin conditions. Typically, sufficient heat is generated for the skin contacting surface 34 to feel warm against the skin of a user (commonly in the range of 37 to 45 degrees Celsius). In an alternative example as shown in Figure 8, a heater 44 may be disposed in potting 30 to provide a greater amount of heat or a more consistent temperature than might be achieved by heat transfer from light sources 28 alone. A radio frequency energy emitter 45 could also be disposed in potting 30 for emitting radio frequency energy for use during treatment. Sensors (not shown) such as temperature sensors and proximity sensors may also be disposed in treatment head 12 for use in controlling operation of device 10.

Skin contacting surface 34 commonly includes formations 46 for optimizing the application of a desired treatment to the skin. It is believed that formations 46 improve performance of device 10 in a number of ways including stretching the skin, opening pores and optimizing the transfer of light, heat and most notably vibration energy from the device 10 to the skin.

Formations 46 may include one or more protrusions 46a (Fig. 8) or depressions 46b (Fig. 7) having a variety of shapes and arrangements such as shown in Figure 7-9 or a combination of each. Formations 46 commonly have a height H or depth D in the range of 0.5-5 mm. Formations 46 may have smooth surfaces or abrasive surfaces depending on the desired treatment. Formations 46, and most commonly depressions 46b, may be sized to fully surround and envelope skin conditions such as warts, blisters or pimples.

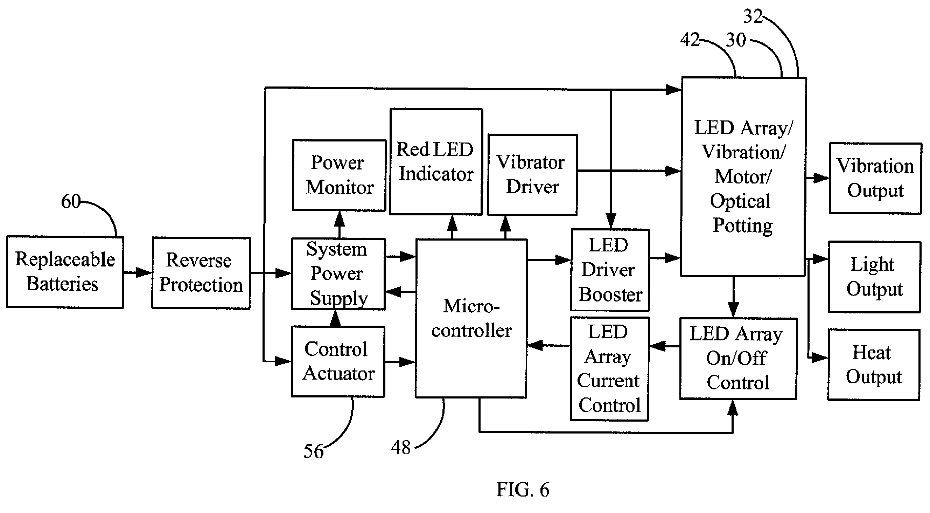

Treatment assembly 20 further includes a vibrator 42 (Figs. 4, 5, 7, and 8), such as, for example, a disc type vibration motor, that engages first PCB 40 on a side opposing the side in contact with rear surface 38 of potting 30 commonly along a common axis A with potting 30. Vibrator 42 is secured to first PCB 40 commonly using an epoxy, welds or other suitable attachment to ensure that vibrations translate to skin contacting surface 34. Vibrator 42 is driven through pulse width modulation under control of a microcontroller 48 (Fig. 6) at a desired operating frequency using a constant vibration driving scheme to adjust to a variable voltage range as batteries deplete overtime from full power to low power. Vibrator 42 may operate at subsonic, sonic or ultrasonic frequencies and most commonly in the range of 5000-25000 Hz.

Commonly, treatment head housing 22 and treatment assembly 20 are formed as one integrated piece. This is accomplished by first soldering the one or more light sources 28 (commonly encapsulated LEDs) to a first side of first PCB 40. First PCB 40 with the attached light sources 28 is then disposed in treatment head housing 22 and light sources 28 and first side of first PCB 40 are encapsulated by depositing potting material through aperture 26 to form potting 30. Sufficient potting is provided to fill cavity 24 and bond first PCB 40, light sources 28 and treatment head housing 22 together into one integrated piece. Vibrator 42 is then disposed on opposing side of first PCB 40 and permanently affixed to first PCB 40 using epoxy, welds or other suitable attachments.

Body 14 (Figs. 1, 2, and 3) includes electric circuitry components aligned along axis A including a control assembly 50 accommodated in a body housing 52. Control assembly 50 includes a second PCB 54 that is operably connected to vibrator 42. A control switch 56 is disposed on the exterior of body housing 52 for controlling operation of the device 10. Control switch 56 may be a simple on/off switch (as shown) or may have additional control settings for controlling operation of the device 10. The additional control settings may include a vibration control for controlling the amount of vibration and/or a light source control for controlling the amount of light emitted by the light source. Control switch 56 is operably connected to microcontroller 48 (Fig. 6) that is disposed on a third PCB 58. Third PCB 58 in turn is connected to a power supply 60, such as one or more replaceable or rechargeable batteries disposed within body housing 52. Body housing 52 is removably connected to treatment head housing 22 to facilitate access to the batteries. Conveniently, batteries for power supply 60 may be disposed in a unitary battery holder 62.

An abrupt or sudden disturbance of device 10 such as falling and hitting a firm surface, may cause electric circuitry components of device 10, such as, for example, PCBs 40, 54 and 58 as well as battery holder 62 to shift axially along, or perpendicularly to axis A (Fig. 4), dislodge and lose physical contact with each other, breaking electrical contact between the components and deeming device 10 inoperable.

Body 14 can also include a bias 64 (Figs. 2 and 4) disposed between divider 66 and battery holder 62 or at any other suitable location along axis A. Bias 64 may be, for example, a spring, a rubber cylinder or similar, be made of any compressible and expandable suitable material and act as a damper, dampening any movement of treatment device 10 electric circuitry components along axis A and pressing device 10 electric circuitry components against each other along axis A so that to prevent such axial or perpendicular movement and the dislodging the components in case of such a disturbance.

Dispenser head 16 can include a dispensing assembly 70, as shown in Fig. 2A, disposed in a dispenser housing 72 covered with a removable cap 74 (Figs, 1, 3, and 4). Dispensing assembly 70 may be any suitable assembly for dispensing a substance for treating a user's skin. For example, dispensing assembly 70 may be a roller (as shown), a stick or a spray nozzle for dispensing treatment substance from a container (not shown) disposed in the dispenser housing 72. The treatment substance may be any topical formulation, typically in liquid, gel or stick form, that may be used for treating specific conditions or that provides other desired effects, therapeutic or otherwise, prior, during or following treatment.

Alternatively and optionally, dispenser head 16 can be hollow or solid and include removable or integrally attached cap 74 only.

Dispenser head 16 may be removably attached to body 14 by a connection 82 disposed on a, for instance by a threaded or snap fit or magnetic connection, so that a different dispenser head 16 may be attached to body 14. Different dispenser head 16 may contain a fresh supply of the treatment substance or an alternate type of treatment substance for desired applications. Alternatively, the substance container may be adapted to receive a refill (not shown) of treatment substance or the substance container may be removable through a hatch (not shown) so that a fresh container may be inserted

Referring to Figure 6, which is a block diagram illustrating the interoperability of certain functional components of the device 10. Power supply 60 provides power to certain functional components while microcontroller 48 controls operation of the functional components. The microcontroller may include a processor and a memory. Some or all of the microcontroller's functionality, such as monitoring power supply 60 and monitoring and control of light source 28, vibrator 42 and (optionally) heater 44, may be programmed into its processor using software that may be stored in its memory. Control switch 56 permits manual control of microcontroller 48 and thus control of the functional components of device 10.

During use, a user may apply a treatment substance to a desired area of the user's skin using dispenser head 16. Dispenser head 16 is conveniently located at one end of device 10 so that the user may then simply turn device 10 around to apply a desired treatment using treatment head 12. The user may then activate control switch 56 to initiate the generation of light, heat and vibration energy. The user then applies the skin contacting surface 34 to the desired area of the user's skin to administer the desired treatment for a desired period of time (two minutes for example). The user may apply a desired amount of pressure against the user's skin to allow formations 46 in skin contacting surface to stretch the skin, open pores and optimize the delivery of the light, heat and vibration energy to the skin. The user may also move device 10 over the skin surface to further stimulate and treatment a larger skin surface area.

The device is intended for use in treating a wide range of conditions that benefit from the application of a combination of light, heat and vibration energies. Typically such conditions are cosmetic or dermatological conditions such as acne, blemishes, warts, cellulite and age related conditions that benefit from the generation of micro-circulation and collagen production. The device may be used for treatment of other conditions such as vaginal warts as well.

While the above description provides examples of one or more processes or devices, it will be appreciated that other processes or devices may be within the scope of the accompanying claims.

## Claims

1. A skin treatment device, comprising:
a potting (30) containing at least one light source (28) for emitting electromagnetic radiation having desired characteristics for treating one or more skin conditions;
a skin contacting surface (34) disposed at one end of the potting (30), the skin contacting surface (34) being adapted for transmitting electromagnetic radiation emitted from the at least one light source (28) to a user's skin;
a vibrator (42) directly or indirectly contacting other end of the potting (30), the vibrator (42) being adapted to cause the skin contacting surface (34) to vibrate; and
a control assembly (50) for controlling operation of the light source (28) and the vibrator (42); wherein
the potting (30) acts to transfer heat generated from the at least one light source (28) to a user's skin through skin contacting surface (34), wherein
the at least one light source (28) and the vibrator (42) are aligned along an axis (A) extending from the one end of the potting (30) to the other end of the potting (30), and wherein the device further comprises
a housing (22) accommodating electric circuitry components aligned along the axis (A) with the light source (28) and the vibrator (42) and a bias (64) axially pressing the electrical circuitry components against each other so as to prevent dislodging of the components in case of a disturbance.

2. The device of claim 1, wherein the skin contacting surface (34) includes one or more formations (46) that are adapted for stretching a portion of a user's skin.

3. The device of claim 2, wherein the formations (46) include at least one depression (46b) sized to fully surround and envelope skin conditions such as warts, blisters or pimples.

4. The device of claim 1, wherein the bias (64) is selected from the group consisting of a spring, a rubber cylinder or any compressible and expandable material that can act as a damper so as to prevent axial or perpendicular movement.

5. The device of claim 1, wherein the at least one light source (28) is an encapsulated LED and the skin contacting surface (34) is defined by an exposed face of the potting (30) and protruding tips of the encapsulated LEDs.

6. The device of claim 1, wherein the at least one light source (28) is an encapsulated LED, and the housing (22) has an aperture (26) at one end thereof and wherein the potting (30) is disposed in a cavity (24) flush with the aperture (26) and tips of the encapsulated LEDs protrude from the aperture (26).

7. The device of claim 1, wherein the potting (30) also includes at least one of a heater (44) and a radio frequency emitter (45).

8. The device of claim 1, wherein the skin contacting surface (34) is defined by a faceplate (36) adapted to transfer light, heat and vibration energy to the skin.

9. The device of claim 6, wherein the potting (30) disposed within the cavity (24) includes a non-skin contacting surface (38) disposed on a side of the potting (30) opposite to the skin contacting surface (34) and connected to a first printed circuit board (PCB) (40).

10. The device of claim 9, wherein first printed circuit board (PCB) (40) includes a plurality of vent holes that allow air to escape when molten material for forming the potting is disposed within the housing (22).

11. Method of manufacture a device according to claim 6 further including one or more printed circuit board (PCB) (40), wherein the method comprises
soldering one or more encapsulated LEDs to a first side of the PCB,
disposing the PCB with encapsulated LEDs in the housing (22),
depositing potting material through the aperture (26) to fill the cavity (24) and bond the PCB, the encapsulated LEDs and the housing (22) into one integrated piece so as to encapsulate the PCB and encapsulated LEDs and form the potting (30).

12. Method according to claim 11, further comprising depositing the vibrator (42) on a second side of the PCB opposing to the first side and fixing the vibrator (42) to the PCB with epoxy or by welding.

## Patentansprüche

1. Eine Vorrichtung zur Behandlung der Haut umfassend:
eine Ummantelung (30) umfassend mindestens eine Lichtquelle (28) zur Ausstrahlung von elektromagnetischer Strahlung mit gewünschten Eigenschaften zur Behandlung von einer oder mehreren Hautbeschwerden;
eine an einem Ende der Ummantelung (30) angeordnete hautkontaktierende Oberfläche (34), wobei die hautkontaktierende Oberfläche (34) angepasst ist, um elektromagnetische Strahlung, die von der mindestens einen Lichtquelle (28) ausgestrahlt wird, an die Haut eines Benutzers zu übertragen;
einen Vibrator (42), der direkt oder indirekt in Kontakt mit einem anderen Ende der Ummantelung (30) kommt, wobei der Vibrator (42) angepasst ist, um eine Vibration der hautkontaktierenden Oberfläche (34) hervorzurufen; und
eine Steueranordnung (50) zur Steuerung des Betriebs der Lichtquelle (28) und des Vibrators (42);
wobei die Ummantelung (30) wirkt, um Hitze, die von der mindestens einen Lichtquelle (28) erzeugt wird, an die Haut eines Benutzers durch die hautkontaktierende Oberfläche (34) zu übertragen, wobei
die mindestens eine Lichtquelle (28) und der Vibrator (42) entlang einer sich von dem einen Ende der Ummantelung (30) bis zum anderen Ende der Ummantelung (30) erstreckende Achse (A) fluchten, und wobei die Vorrichtung weiterhin folgendes umfasst
ein Gehäuse (22), welches Bestandteile von elektrischen Schaltungen unterbringt, die entlang der Achse (A) mit der Lichtquelle (28) und dem Vibrator (42) fluchten, und ein Vorspannungsmittel (64), welches die Bestandteile von elektrischen Schaltungen axial gegeneinanderdrückt, um das Entfernen der Bestandteile im Falle von einer Störung zu vermeiden.

2. Die Vorrichtung des Anspruchs 1, wobei die hautkontaktierende Oberfläche (34) eine oder mehrere Gestaltungen (46) umfasst, welche angepasst sind, um einen Teil der Haut eines Benutzers zu strecken.

3. Die Vorrichtung des Anspruchs 2, wobei die Gestaltungen (46) mindestens eine Vertiefung (46b) umfassen, deren Größe das vollständige Umgeben und Einhüllen von Hautbeschwerden wie etwa Warzen, Blasen oder Pickel ermöglichen.

4. Die Vorrichtung des Anspruchs 1, wobei das Vorspannungsmittel (64) aus der Gruppe bestehend aus einer Feder, einem Gummizylinder oder einem komprimierbaren und ausdehnbaren Material ausgewählt ist, welches als Puffer zur Hinderung von axialen bzw. senkrechten Bewegungen wirken kann.

5. Die Vorrichtung des Anspruchs 1, wobei die mindestens eine Lichtquelle (28) eine gekapselte LED ist und die hautkontaktierende Oberfläche (34) durch eine ausgesetzte Seite der Ummantelung (30) und herausragende Spitzen der gekapselten LEDs definiert ist.

6. Die Vorrichtung des Anspruchs 1, wobei die mindestens eine Lichtquelle (28) eine gekapselte LED ist und das Gehäuse (22) eine Öffnung (26) ein einem Ende davon hat und wobei die Ummantelung (30) in einer Höhlung (24) bündig mit der Öffnung (26) angeordnet ist und Spitzen der gekapselten LEDs aus der Öffnung (26) herausragen.

7. Die Vorrichtung des Anspruchs 1, wobei die Ummantelung (30) ferner mindestens einen von einem Heizkörper (44) und einem Radiofrequenzsender (45) umfasst.

8. Die Vorrichtung des Anspruchs 1, wobei die hautkontaktierende Oberfläche (34) durch eine Abdeckung (36) definiert ist, welche angepasst ist, um Licht, Hitze und Schwingungsenergie an die Haut zu übertragen.

9. Die Vorrichtung des Anspruchs 6, wobei die innerhalb der Höhlung (24) angeordnete Ummantelung (30) eine nicht-hautkontaktierende Oberfläche (38) umfasst, welche auf einer gegenüber der hautkontaktierenden Oberfläche (34) liegenden und mit einer ersten Leiterplatte - PCB - (40) verbundenen Seite der Ummantelung (30) angeordnet ist.

10. Die Vorrichtung des Anspruchs 9, wobei die erste Leiterplatte (40) eine Vielzahl von Entlüftungsöffnungen umfasst, welche die Entlüftung ermöglichen, wenn geschmolzenes Material zur Bildung der Ummantelung innerhalb des Gehäuses (22) abgegeben wird.

11. Verfahren zur Herstellung von einer Vorrichtung nach Anspruch 6, weiterhin umfassend eine oder mehrere Leiterplatten - PCB - (40), wobei das Verfahren folgendes umfasst
das Löten von einer oder mehreren gekapselten LEDs an einer ersten Seite der PCB,
das Anordnen der PCB mit gekapselten LEDs innerhalb des Gehäuses (22),
das Einlegen von Vergussmaterial durch die Öffnung (26), um die Höhlung (24) zu füllen und die PCB, die gekapselten LEDs und das Gehäuse (22) als ein einziges Stück zu binden, um die PCB und die gekapselten LEDs einzukapseln und die Ummantelung (30) auszubilden.

12. Verfahren nach Anspruch 11, weiterhin umfassend das Einlegen des Vibrators (42) auf einer zweiten Seite der PCB, die gegenüber der ersten Seite liegt und das Befestigen des Vibrators (42) an der PCB mittels Epoxid oder durch Schweißarbeit.

## Revendications

1. Un dispositif de traitement de la peau, comprenant :
un enrobage (30) contenant au moins une source de lumière (28) pour émettre du rayonnement électromagnétique ayant des caractéristiques désirées pour traiter une ou plusieurs conditions de la peau ;
une surface (34) en contact avec la peau disposée dans une extrémité de l'enrobage (30), étant la surface (34) en contact avec la peau adaptée pour transmettre du rayonnement électromagnétique émet par l'au moins une source de lumière (28) à la peau d'un utilisateur ;
un vibrateur (42) en contact direct ou indirect avec une autre extrémité de l'enrobage (30), étant le vibrateur (42) adapté pour entraîner une vibration de la surface (34) en contact avec la peau ; et
un assemblage de contrôle (50) pour contrôler l'opération de la source de lumière (28) et le vibrateur (42) ; dans lequel
l'enrobage (30) agit en transférant de la chaleur générée par l'au moins une source de lumière (28) à la peau d'un utilisateur au travers de la surface (34) en contact avec la peau, dans lequel
l'au moins une source de lumière (28) et le vibrateur (42) sont alignés le long d'un axe (A) s'étendant de l'une extrémité de l'enrobage (30) à l'autre extrémité de l'enrobage (30), et dans lequel le dispositif comprend en outre
un boîtier (22) logeant des composants de circuits électriques alignés le long d'un axe (A) avec la source de lumière (28) et le vibrateur (42) et un moyen de précontrainte (64) pressant axialement les composants de circuits électriques les uns contre les autres de façon à prévenir le délogement des composants en cas de dérangement.

2. Le dispositif de la revendications 1, dans lequel la surface (34) en contact avec la peau inclut une ou plusieurs formations (46) qui sont adaptées pour étirer une partie de la peau d'un utilisateur.

3. Le dispositif de la revendication 2, dans lequel les formations (46) incluent au moins un renfoncement (46b) dimensionné de façon à entourer et envelopper complètement des conditions de la peau telles que des verrues, des ampoules ou des boutons.

4. Le dispositif de la revendication 1, dans lequel le moyen de précontrainte (64) est choisi dans le groupe constitué d'un ressort, d'un cylindre de caoutchouc ou d'un matériau compressible et expansible quelconque qui puisse agir en tant que tampon pour prévenir le mouvement axial ou perpendiculaire.

5. Le dispositif de la revendication 1, dans lequel l'au moins une source de lumière (28) est un DEL encapsulé et la surface (34) en contact avec la peau est définie par une face exposée de l'enrobage (30) et des pointes faisant saillie des DEL encapsulés.

6. Le dispositif de la revendication 1, dans lequel l'au moins une source de lumière (28) est un DEL encapsulé, et le boîtier (22) a une ouverture (26) dans une extrémité de celui-ci et dans lequel l'enrobage (30) est disposé dans une cavité (24) alignée avec l'ouverture (26) et des pointes des DEL encapsulés font saillie par rapport à l'ouverture (26).

7. Le dispositif de la revendication 1, dans lequel l'enrobage (30) inclut aussi au moins un d'entre un appareil de chauffage (44) et un émetteur à fréquence radio (45).

8. Le dispositif de la revendication 1, dans lequel la surface (34) en contact avec la peau est définie par un plateau (36) adapté pour transférer de la lumière, de la chaleur et de l'énergie de vibration à la peau.

9. Le dispositif de la revendication 6, dans lequel l'enrobage (30) disposé dans la cavité (24) inclut une surface (38) qui n'est pas en contact avec la peau disposée sur un côté de l'enrobage (30) opposé à la surface (34) en contact avec la peau et reliée à une première carte de circuit imprimé - PCB - (40).

10. Le dispositif de la revendication 9, dans lequel la première carte de circuit imprimé (40) inclut une pluralité de trous de ventilation qui permettent l'échappement d'aire lorsque du matériau fondue destiné à la formation de l'enrobage est disposé dans le boîtier (22).

11. Procédé de fabrication d'un dispositif selon la revendication 6 incluant en outre une ou plusieurs cartes de circuit imprimé - PCB - (40), dans lequel le procédé comprend
braser un ou plusieurs DEL encapsulés à un premier côté de la PCB,
disposer la PCB avec des DEL encapsulés dans le boîtier (22),
déposer du matériau d'enrobage au travers de l'ouverture (26) afin de remplir la cavité (24) et de lier la PCB, les DEL encapsulés et le boîtier (22) en formant une pièce intégrée afin d'encapsuler la PCB et les DEL encapsulés et de former l'enrobage (30).

12. Procédé selon la revendication 11, comprenant en outre déposer le vibrateur (42) sur un deuxième côté de la PCB opposé au premier côté et fixer le vibrateur (42) à la PCB moyennant une résine époxy ou par soudage.
